# EUROPEAN PATENT SPECIFICATION

(11) **EP 1 943 967 B1**
(45) Date of publication and mention of the grant of the patent: **11.01.2012**
(21) Application number: 07021243.6
(22) Date of filing: 31.10.2007
(51) Int. Cl.: A61B 17/17, A61B 19/00

(54) **Centering device for orthopaedic surgery**
Zentriervorrichtung für die orthopädische Chirurgie
Dispositif de centrage pour une chirurgie orthopédique

(30) Priority: 11.01.2007 IT MI20070032
(43) Date of publication of application: 16.07.2008
(73) Proprietor: IRMA DI GOSIO & C. S.r.l., 25125 Brescia (IT)
(72) Inventor: Claudio, Enrico, 25100 Brescia (IT)
(74) Representative: Lecce, Giovanni

(56) References cited:
- JP-A- 2000 312 680
- JP-A- 2003 245 283
- US-A- 5 649 930
- US-A1- 2002 007 188
- US-A1- 2005 131 418
- US-A1- 2005 177 171

## Description

This invention relates to a centring device for orthopaedic surgery.

More in particular, this invention relates to a centring device, intended for mobile units with brilliance amplifiers, usable in operations that envisage intramedullary nailing.

It is known that at present orthopaedic surgery uses several techniques, among which intramedullary nailing. This procedure is carried out on diaphyseal long bone fractures, inserting a nail of the same diameter as the intramedullary canal inside the fractured bone, starting from the epiphysis of the same bone and using dedicated instruments.

Operatively, the surgeon makes a hole at the apex of the fractured bone and inserts the intramedullary nail; the latter is therefore made integral with the same bone by through screws. A so-called "compass" is used to insert such screws in suitable positions, which is mounted at the apex of the intramedullary nail, so as to reduce the risk on failure.

However, this traditional solution has considerable drawbacks.

In fact, the farther the screws from the point of insertion of the nail or "apex" are, the longer the compass arm is, so it is more difficult to centre the hole to insert the distal screw.

Due to these drawbacks, the procedure often implies repeated attempts, thus increasing the surgery time that the orthopaedist carries out with the aid of the so-called brilliance amplifier; however, the use of such amplifier exposes the doctor - as well as the patient - to a considerable amount of radiations.

Also the use of the brilliance amplifier, in any case, does not ensure a safe centring; the technique adopted to this end traditionally envisage centring with such positioning of the amplifier as to obtain an image of the nail hole as much as possible round, reasonably assuming that the same is impinged by the X rays according to a perpendicular incidence. This is only partly true, considering that the source of X rays is dot-like and that then, only the centre of the X ray bundle can be considered as incident or central ray and is the one that falls exactly at the centre of the brilliance amplifier, while the others are diverging.

As a consequence, if the nail hole is not represented exactly at the centre of the monitor, which is the display means connected to the brilliance amplifier, the image obtained is not usable for a safe centring.

US 2005/0131418 discloses an intramedullary nail drill guide for a radiation device comprising an X ray source, a receptor and a C-arm, in which the drill guide can be adjustably mounted to any portion of the radiation device and is swivelling in respect of the X ray beam axis. The reference is not concerned with a centring device.

The object of this invention is to obviate the drawbacks mentioned hereinabove.

More in particular, the object of this invention is to provide a centring device for orthopaedic surgery requiring intramedullary nailing as claimed in claim 1, suitable for allowing easy and accurate detection of the hole(s) for inserting the distal screw(s) in the nail previously inserted in the bone.

A further object of the invention is to provide a device as defined above which allows the orthopaedist to carry out said hole centring in any operating condition, without the risk of being exposed to X rays.

Last but not least, an object of the invention is to provide a centring device as defined above which allows optimising the action of the brilliance amplifier for a safe centring of the holes of the intramedullary nail.

A further object of this invention is to provide the users with a centring device for orthopaedic surgery suitable for ensuring a high level of resistance and reliability over time, also such as to be easily and inexpensively constructed.

These and other objects are achieved by the centring device for orthopaedic surgery as claimed in claim 1. Additional advantageous features are recited in the dependent claims.

The construction and functional features of the centring device for orthopaedic surgery of the invention shall be better understood from the following detailed description, wherein reference is made to the annexed drawings showing a preferred and non-limiting embodiment thereof, and wherein:

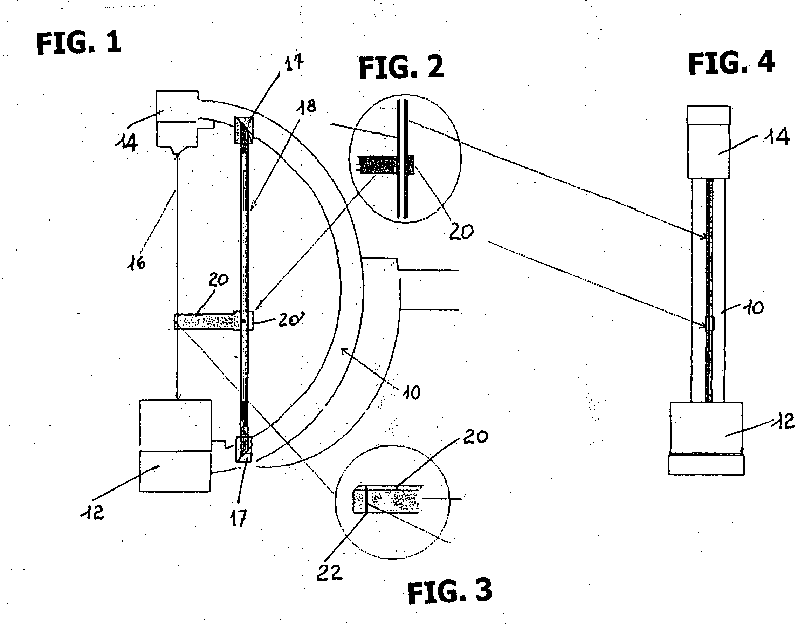

figure 1 schematically shows a side view of the centring device for orthopaedic surgery of this invention combined with a unit provided with brilliance amplifier;
figure 2 shows an enlarged detail of figure 1;
figure 3 shows a further enlarged detail of figure 1;
figure 4 schematically shows a front view of the same centring device.

With reference to the above figures, the centring device for orthopaedic surgery of this invention is combined with the semi-circular "C" shaped metal frame 10 of the mobile unit, known per se, comprising a brilliance amplifier 12 facing an X-ray tube 14.

The semi-circular "C" shaped frame 10 is typically mounted on guides, so that the central beam or incident beam, schematised with 16, of the X-ray tube 14 is always perpendicular to the centre of the brilliance amplifier 12 that detects the image thereof. This is to allow the radiologist to orientate the same amplifier in any direction, so as to detect the images of the anatomic part being examined according to all possible angles, according to the orthopaedic surgeon's needs.

The centring device of this invention comprises at least one fixed bar 18, preferably of steel and mounted inside two fixed supports 17 firmly anchored by screws and/or welding or the like to the semi-circular "C" shaped frame 10; said fixed bar 18 is fixed to the semi-circular "C" shaped frame 10 so as to be parallel to the incident beam 16 of the bundle of beams emitted by the X-ray tube 14. The fixed bar 18, moreover, can be removed for cleaning and sterilising.

A mobile bar 20 slides on the fixed bar 18, made of radio-transparent material such as for example aluminium; the mobile bar 20 is provided with a collar shaped end 20' for coupling with the fixed bar 18 and, relative to the latter, it is oriented orthogonally in the direction of the open front of the semi-circular "C" shaped frame 10.

The same mobile bar 20 extends in the semi-circular "C" shaped frame 10 up to passing beyond the line defined by the incident beam 16 and in the proximity of the free end thereof, it is provided with a through hole 22 transversally extended and made in precise alignment with the same incident beam 16.

Owing to the parallelism between fixed bar 18 and incident beam 16, during operating step the hole 22 of the mobile bar 20 is constantly and precisely crossed by said incident beam 16, irrespective of the position thereof along the fixed bar 18.

Given these features of the centring device of this invention, the incident beam 16 is used as pointer, since it allows checking the position of the hole of the intramedullary nail arranged inside the bone exactly.

In fact, through simple manoeuvres, using the articulations each mobile unit is provided with, the radiologist can orientate the beam of X rays in any spatial direction, up to obtain the hole of the intramedullary nail on the monitor exactly at the centre and overlapped to hole 22 of the mobile bar 20.

Once the hole 22 of the mobile bar 20 has been matched with the hole of the intramedullary nail, the surgeon can safely insert the drill bit in said hole 22 and drill the bone to insert the screw therein; the exact position for drilling is given by the incident beam 16 of the brilliance amplifier 12 without risks of errors.

To make the drilling, the surgeon advantageously uses bushes of suitable diameter, in relation to the drill bit to be used, previously inserted in the hole 22 of the mobile bar 20, for making a hole of suitable diameter; this is in order to define a dimensionally precise guiding channel for the drill bit and prevent it from arranging angularly relative to the incident beam 16.

The fixed 18 and mobile 20 bars can optionally be composed of sections or tubular elements of any section.

As can be noticed from the above, the advantages achieved by the invention are clear.

The centring device of this invention allows precisely and quickly locating the position of the hole(s) on the intramedullary nail inserted in the bone, thus providing the surgeon with precise indications for drilling and considerably reducing the overall operation times. Moreover, the possibility of using brilliance amplifiers without exposing the operators in the operating room to X rays and drastically reducing the amount of X rays absorbed by the patient is particularly advantageous.

Moreover, the centring device for orthopaedic surgery of this invention is advantageously usable in the so-called "mini-open" technique used for internal fixing devices, for removing the synthesis devices with mini-invasive techniques, for the spatial detection of radio-opaque foreign bodies and in the centring for biopsies; this implies that the device of the invention may be used by the orthopaedic surgeon for precisely locating an anatomic structure or a radio-opaque object before cutting into the skin.

## Claims

1. A centering device for orthopedic surgery, usable during operations that envisage intramedullary nailing, applied to a semi-circular "C" shaped metal frame (10) provided with a brilliance amplifier (12) and an X-ray tube (14) emitting a bundle of X rays towards said brilliance amplifier (12), said device comprising:
at least one fixed bar (18) secured to said "C" shaped frame (10), and mobile means being slidingly movable on said fixed bar (18), the centering device **characterized in that** said mobile means is defined by a mobile bar (20) oriented orthogonally relative to the fixed bar (18) in the direction of the open front of the semi-circular "C" shaped frame (10), the mobile bar being provided with a through hole (22) made in proximity of the free end of said mobile bar and transversally extended, said hole (22) being constantly and precisely crossed by the central beam of said X-ray bundle (16) irrespective of the position thereof along the fixed bar (18), both ends of said fixed bar (18) being mounted inside two fixed supports (17) firmly anchored to said "C" shaped frame (10) whereby said fixed bar (18) is parallel to the central beam (16) .

2. A centering device according to claim 1, **characterized in that** the hole (22) of the mobile bar (20) is adapted to receive bushes of different diameters to act as a guide for a drill bit that makes the drilling for fixing the intramedullary nail.

3. A centering device according to claim 1 or 2, **characterized in that** said mobile bar (20) is arranged relative to the fixed bar (18) with its free end longitudinally extending so as to protrude beyond the line defined by said incident beam (16).

4. A centering device according to claim 3, **characterized in that** said mobile bar (20) is made of radio-transparent material.

## Patentansprüche

1. Eine Zentriervorrichtung für die orthopädische Chirurgie, die bei Operationen einsetzbar ist, bei denen die Marknagelung vorgesehen ist, die auf einen halbrunden "C"-förmigen Metallrahmen (10) angebracht ist, der mit einem Helligkeitsverstärker (12) und einer Röntgenröhre (14) ausgestattet ist, die ein Bündel von Röntgenstrahlen in Richtung des besagten Helligkeitsverstärkers (12) emittiert, wobei die Vorrichtung Folgendes umfasst:
mindestens eine feste Stange (18), die an dem besagten "C"-förmigen Rahmen befestigt ist und ein bewegliches Mittel, das sich auf der festen Stange (18) durch Verschieben bewegen lässt, wobei die Zentriervorrichtung **dadurch gekennzeichnet ist, dass** dieses besagte bewegliche Mittel durch eine bewegliche Stange (20) definiert ist, die orthogonal zu der festen Stange (18) in Richtung der offenen Vorderseite des halbrunden "C"-förmigen Rahmens (10) ausgerichtet ist, wobei die bewegliche Stange mit einem Durchgangsloch (22) versehen ist, das in der Nähe des freien Endes der besagten beweglichen Stange erstellt und quer erweitert ist, wobei das besagte Loch (22) kontinuierlich und präzise von dem zentralen Strahl (16) des besagten Bündels von Röntgenstrahlen durchquert wird, unabhängig von dessen Position entlang der festen Stange (18),
wobei die beiden Enden der besagten festen Stange (18) im Inneren von zwei festen Halterungen (17) angebracht sind, die an dem besagten "C"-förmigen Rahmen (10) fest verankert sind, wobei diese feste Stange (18) parallel zu dem zentralen Bündel angeordnet ist (16).

2. Eine Zentriervorrichtung nach Anspruch 1, die **dadurch gekennzeichnet ist, dass** das Loch (22) der beweglichen Stange (20) geeignet ist, Buchsen mit unterschiedlichen Durchmessern aufzunehmen, um als Führung für einen Bohrer zu dienen, der die Bohrungen für die Befestigung der Marknägel erstellt.

3. Eine Zentriervorrichtung nach Anspruch 1 oder 2, die **dadurch gekennzeichnet ist, dass** die besagte bewegliche Stange (20) zu der festen Stange (18) sich der Länge nach erstreckend so angeordnet ist, dass ihr freies Ende über die Linie herausragt, die durch den besagten einfallende Strahl definiert ist (16).

4. Eine Zentriervorrichtung nach Anspruch 3, die **dadurch gekennzeichnet, dass** die besagte bewegliche Stange (20) aus funkdurchlässigem Material besteht.

## Revendications

1. Un dispositif de centrage pour la chirurgie orthopédique, utilisable pendant les opérations qui envisagent le clouage intramédullaire, appliqué à une ossature métallique semi-circulaire en forme de « C » (10) muni d'un amplificateur de brillance (12) et un tube de rayons X (14) émettant un faisceau de rayons X en direction dudit amplificateur de brillance (12), ledit dispositif comprenant :
au moins une barre fixe (18) solidaire à ladite ossature en forme de « C » et les moyens mobiles étant mobiles en coulissement sur ladite barre fixe (18), le dispositif de centrage **caractérisé en ce que** lesdits moyens mobiles sont définis par une barre mobile (20) orientée orthogonalement par rapport à la barre fixe (18) en direction du front de l'ouverture de l'ossature semi-circulaire en forme de « C » (10), la barre mobile étant munie d'un trou à travers (22) fait à proximité de l'extrémité libre de ladite barre mobile et transversalement étendu, ledit trou (22) étant constamment et précisément traversé par le faisceau central (16) dudit rayon X, indépendamment de la position de celui-ci le long de la barre fixe (18),
les deux extrémités de ladite barre fixe (18) étant montée à l'intérieur de deux supports fixes (17) solidement ancrée à ladite ossature en forme de « C » (10) où ladite barre fixe (18) est parallèle au faisceau central (16).

2. Un dispositif de centrage selon la revendication 1, **caractérisé en ce que** le trou (22) de la barre mobile (20) est adapté pour recevoir des bagues de différents diamètres servant comme un guide pour un foret qui fait le perçage pour la fixation du clou intramédullaire.

3. Un dispositif de centrage selon la revendication 1 ou 2, **caractérisé en ce que** ladite barre mobile (20) est disposée par rapport à la barre fixe (18) avec son extrémité libre qui s'étend longitudinalement de façon à faire saillie au-delà de la ligne définie par ledit faisceau incident (16).

4. Un dispositif de centrage selon la revendication 3, **caractérisé en ce que** ladite barre mobile (20) est faite de matériel radio-transparent.
